(19) Europäisches Patentamt
European Patent Office
Office européen des brevets

(11) Publication number: **0 118 979**
**A1**

(12) # EUROPEAN PATENT APPLICATION

(21) Application number: 84300444.1

(22) Date of filing: 25.01.84

(51) Int. Cl.³: C 12 N 11/02
C 12 N 11/00, C 12 P 19/24

(30) Priority: 14.02.83 GB 8304069

(43) Date of publication of application: 19.09.84 Bulletin 84/38

(84) Designated Contracting States: DE FR GB NL

(71) Applicant: IMPERIAL CHEMICAL INDUSTRIES PLC
Imperial Chemical House Millbank
London SW1P 3JF(GB)

(72) Inventor: Corcoran, Eugene Gerard
Blackpool The Spa
Tralee Co. Kerry(IE)

(74) Representative: Aufflick, James Neil et al,
Imperial Chemical Industries PLC Legal Department: Patents PO Box 6 Bessemer Road
Welwyn Garden City Herts, AL7 1HD(GB)

(54) **Production of immobilised enzymes.**

(57) An improved method for immobilizing enzymes in flocculated whole microbial cells in which the cells are treated with a water-insoluble binding agent, particularly casein, and thereafter with a polycationic flocculating agent and a polyanionic flocculating agent, immobilised enzymes in aggregate particles produced by the method and a process for performing an enzyme-catalysed reaction using the particles. Preferably the enzyme is flucose isomerase and the process is for the conversion of glucose to fructose.

EP 0 118 979 A1

Production of immobilised enzymes

This invention relates to a method for the production of an immobilised enzyme, to an immobilised enzyme produced by the method and to a process for performing an enzyme catalysed reaction using an immobilised enzyme. In particular the invention relates to a method for the production of immobilised glucose isomerase, to immobilised glucose isomerase produced by the method and to a process for isomerizing glucose to fructose using immobilised glucose isomerase.

Fructose is considerably sweeter than its readily available isomer glucose and there is increasing demand for fructose-containing syrups for use as sweeteners in the manufacture of foodstuffs such as confectionary and in other ways. During the past 20 years increasing attention has been given to processes for isomerizing glucose to fructose. The alkaline isomerization of glucose to fructose has been extensively studied by many workers but the yields obtained by this means are unsatisfactory due to the formation of significant amounts of unwanted by-products. The enzymic isomerisation which is now generally preferred is based upon the discovery that certain microorganisms produce the enzyme glucose isomerase which catalyses the isomerization of glucose to fructose. Patent Specifications relating to the enzymic isomerisation of glucose to fructose include UK Specifications 1103394, 1328970 and 1451273 in which the enzyme is derived from Streptomyces, Arthrobacter and Curtobacterium strains respectively and our UK Specification 1492258 which discloses the

production of glucose isomerase by continuous fermentation.

To be successful an enzymic process for isomerizing glucose to fructose requires a simple but effective means for immobilising glucose isomerase so that it may be used repeatedly in either continuous or batch processes. Most of the microorganisms known to produce glucose isomerase involve intracellular enzyme. Consequently in immobilising glucose isomerase most interest has concentrated upon processes in which glucose isomerase is immobilised in microbial cells.

US Patent 4060456 describes a continuous process for isomerizing glucose to fructose in the presence of glucose isomerase-containing microbial cells which have been aggregated by a prior treatment with 0.5 to 50 percent by weight based upon the wet weight of the cells of a polyelectrolyte flocculating agent. For the effective commercial operation of the process of US Patent 4060456 it has been found to be desirable to use a combination of polycationic and polyanionic flocculating agents with "Primafloc C-7" (Rohm & Haas of Philadelphia, Pa., USA) being by far the most effective polycationic flocculating agent. The great superiority of "Primafloc C-7" to other polycationic flocculating agents in the successful commercial operation of the process of US Patent 4060456 is a considerable disadvantage to the process. The effective dependence for successful operation of the process upon "Primafloc C-7" which is thus produced renders the process inflexible in that it becomes dependent upon readily available supplies of "Primafloc C-7". When supplies of this reagent are interrupted for any reason, it is not possible to change to an alternative polycationic flocculating agent without a considerable decrease in the effectiveness of the process.

According to a particular aspect of the present invention we provide a method for the production of immobilised glucose isomerase in which glucose isomerase-containing microbial cells are formed into an aggregate by treating the cells with a water insoluble binding agent having a particle size within the range 10 - 500 $\mu$ diameter and thereafter with a polycationic

flocculating agent and a polyanionic flocculating agent followed by recovery of aggregate particles.

Further according to the particular aspect of the present invention we provide immobilised glucose isomerase in aggregate particles comprising glucose isomerase-containing microbial cells, said particles being formed by treating the cells with a water insoluble binding agent having a particle size within the range 10 - 500 μ diameter and thereafter with a polycationic flocculating agent and a polyanionic flocculating agent followed by recovery of aggregate particles.

Further according to the particular aspect of the present invention we provide a process for the isomerization of glucose to fructose in the presence of aggregate particles comprising glucose isomerase-containing microbial cells, the aggregate particles having been formed by treating the cells with a water insoluble binding agent having a particle size within the range 10 - 500 μ diameter and thereafter with a polycationic flocculating agent and a polyanionic flocculating agent followed by recovery of aggregate particles, the process comprising contacting the aggregate particles with a glucose solution at a temperature in the range $50^{\circ}$ to $90^{\circ}C$ and at a pH in the range 6 to 10 to effect isomerization of at least a portion of the glucose in the solution and recovering a syrup containing glucose and fructose.

The invention is also applicable to the immobilisation of enzymes generally.

Therefore also according to the present invention we provide a method for the production of an immobilised enzyme in which microbial cells containing the enzyme are formed into an aggregate by treating the cells with a water insoluble binding agent having a particle size within the range 10 - 500 μ diameter and thereafter with a polycationic flocculating agent and a polyanionic flocculating agent followed by recovery of aggregate particles.

Also according to the present invention we provide an

immobilised enzyme in aggregate particles comprising microbial cells containing the enzyme, said particles being formed by treating the cells with a water insoluble binding agent having a particle size within the range 10 - 500 µ diameter and thereafter with a polycationic flocculating agent and a polyanionic flocculating agent followed by recovery of aggregate particles.

Also according to the present invention we provide a process for performing an enzyme-catalysed reaction in the presence of aggregate particles comprising microbial cells containing the enzyme which catalyses the reaction, the aggregate particles having been formed by treating the cells with a water insoluble binding agent having a particle size within the range 10 - 500 µ diameter and thereafter with a polycationic flocculating agent and a polyanionic flocculating agent followed by recovery of aggregate particles, the process comprising contacting the aggregate particles with a reactant under conditions suitable for performing the enzyme catalysed reaction and recovering a product of the reaction.

In its application to the immobilisation of enzymes generally in addition to the immobilisation of glucose isomerase we believe that the invention is particularly applicable to the immobilisation of enzymes useful in the food industry such as lactase and amyloglucosidase. Other enzymes which can be immobilised include the enzyme cyanide hydrolase (also known as formamide-hydrolyase and cyanide hydratase). This last enzyme catalyses the step in the degradation of cyanide in which cyanide is converted into formamide by the reaction:-

$$HCN + H_2O \longrightarrow HCO\ NH_2.$$

The use of this enzyme in the treatment of cyanide-containing effluent is described in our published European Patent Specification No. 61249.

In the method of the invention the binding agent assists in binding together flocculated microbial cells into an aggregate having good structural integrity. In the formation of aggregates of cells containing glucose isomerase using casèin as the binding

agent, we have found that the products have higher enzyme activities per gm dry weight of cells than are shown by products made by conventional methods using polycationic and polyanionic flocculating agents. We believe that this finding is the result of the casein binding agent reducing diffusion problems in the aggregate. We also believe that a similar reduction of diffusion problems with a resulting increase in enzyme activity can also be achieved with enzymes which exhibit more serious diffusion problems such as the enzyme cyanide hydrolase. A wide range of compounds are suitable for use as binding agents in the method of the invention including casein and other proteins such as serum albumin which are in a water insoluble form. Suitable proteins may be naturally insoluble or may be treated, e.g. by denaturation, to render them insoluble. However the compound which is particularly preferred as a binding agent is casein. The binding agent is added in particulate form, the particles being in the range 10 - 500 μ in diameter.

The polycationic and polyanionic flocculating agents used in the present invention are polyelectrolytes preferably being water soluble polymeric substances containing monomeric units which have polar or ionisable groups. The polycationic agents which are suitable will generally contain quaternary ammonium, sulphonium or phosphonium groups including the protonated forms of polyethylenimine and polyvinylpyridine. Further examples of polyelectrolytes useful as polycationic flocculating agents may be found in the comprehensive review by M F Hoover, J Macromol. Sci. - Chem. A4(6), pp 1327 - 1417 (1970). Polyelectrolytes suitable for use as polyanionic flocculating agents will generally contain carboxylic, sulphonic or phosphonic acid groups and examples of such compounds include polyacrylic acid, polystyrenesulphonic acid, polyvinylphosphonic acid, carboxymethylcellulose, alginic acid and pectic acid. Specific polyelectrolytes useful as polycationic flocculating agents in the invention include "Primafloc C-7" (Rohm & Haas of Philadelphia, Pa., USA), "Decapol X3000" (Fospur Ltd) and "Fospur 733/DPL" (Fospur Ltd). Specific

polyelectrolytes useful as polyanionic flocculating agents in the invention include "Primafloc A-10" (Rohm & Haas of Philadelphia, Pa., USA), "Magnafloc 575" and "Viscalex V92" (Allied Colloids Corp).

Most of the polycationic flocculating agents which are commonly commercially available contain quaternary ammonium groups. "Primafloc C-7", the hitherto preferred polycationic agent for the process of USP 4060456, contains a quaternary ammonium group in which the nitrogen atom is included in a heterocyclic ring. Most other commonly available polycationic flocculating agents however contain quaternary ammonium groups in which the nitrogen atoms are not included in heterocyclic rings. In theory at least a nitrogen atom included in a heterocyclic ring will be less sterically hindered than a chain or terminal nitrogen atom. We believe that the invention of the present application enables polycationic flocculating agents having chain or terminal nitrogen atoms in their quaternary ammonium groups to be used successfully in the process of USP 4060456 or in similar processes.

The method of the invention can usefully be applied to bacterial cells containing glucose isomerase which are produced by cultivating, cells e.g. of strains of the genera <u>Arthrobacter</u> or <u>Curtobacterium</u>, in a fermenter as described in UK Patent Specifications 1328970 or 1451273 respectively. Culture containing the cells is removed from the fermenter and passes into a large holding tank. The binding agent, preferably casein, is added to the culture leaving the fermenter or in the holding tank. The polycationic and polyanionic flocculating agents are then added to the culture treated with the binding agent. Preferably the polycationic agent is added first with stirring causing flocculation of the cells in the culture to occur. The polyanionic flocculating agent is then added with stirring causing the flocculated cells to be compacted into an aggregate. The flocculated cellular product is then filtered, washed with water and dried in a fluidised bed drier to a solids content of about 40%. This stage is termed primary drying. The product after primary drying is still

wet to touch but excess moisture has been removed. The primary dried product is passed through an extruder having an aperture size of for example 1.5 mm. It is then further dried in a secondary drier to give a product containing approximately 93% to 98% solids. This product is then milled and sieved to give a final product comprising aggregate particles approximately 10 to 30 mesh in size.

The binding agent, particularly casein, is suitably added to the culture in an amount greater than 5% (preferably 20% to 50%) of the dry weight of the cells in the culture. On account of the addition of the binding agent it is necessary to add larger amounts of the polycationic and polyanionic flocculating agents than are conventionally added in for example the process of USP 4060456. The amount of the polycationic flocculating agent which is added is suitably in the range 0.02 g to 0.5 g per g dry weight of cells in the culture plus added binding agent combined. The amount of the polyanionic flocculating agent which is added is suitably in the range 0.004 g to 0.5 g per g dry weight of cells in the culture plus added binding agent combined.

Products of enhanced structural integrity may be obtained by the addition of a suitable adhesive material to the cell containing culture after the addition thereto of the binding agent and the polycationic and polyanionic flocculating agents. A compound suitable for use as an adhesive material is one having a molecular weight not greater than 1000 and having a plurality of functional groups, for example a dialdehyde. The preferred adhesive material is glutaraldehyde which is suitably added to a concentration in the range 0.2% to 0.5% on the final concentration of the culture after all additions.

In the method of the invention for the production of an immobilised enzyme such as glucose isomerase the binding agent and the polycationic and polyanionic flocculating agents are preferably added to a suspension of cells at a temperature in the range $10^{o}$ to $40^{o}$ and at a pH in the range 5.0 to 9.5. The polycationic and polyanionic flocculating agents are preferably added in the form of 1

to 2% aqueous solutions. The amounts of polycationic and polyanionic flocculating agents which are used are influenced by the presence of other materials in the cell-containing medium. Thus the flocculation of washed enzyme-containing microbial cells requires slightly less of the flocculating agents than cells which are flocculated directly in culture issuing from a fermenter. The particular microorganism involved will also influence the amounts of polycationic and polyanionic flocculating agents required.

The microbial cells of the immobilised glucose isomerase enzyme aggregates of the invention may be derived from any suitable strain of microorganism. Preferred microorganism strains are generally bacterial strains. Examples of suitable strains which may be mentioned include strains of species of the genera Streptomyces, Arthrobacter and Curtobacterium. Preferred strains of the genus Arthrobacter include those deposited in the Agricultural Research Culture Collection (NRRL), 1815 North University Street, Peoria, Illinois 61604, USA, as strains NRRL B-3724 to B-3728 inclusive. Reference is made to these strains in US Patent 3645848 and corresponding UK Patent 1328970. A particularly preferred strain is strain NRRL B-3728. The microorganism selected as the source of the glucose isomerase is cultured in an appropriate nutrient medium to induce formation of the enzyme. At the end of the fermentation period the isomerase-containing cells are recovered and immobilised.

Preferably glucose isomerase is immobilised by treating the isomerase-containing cells with binding and flocculating agents whilst the cells remain in the culture medium in which they were grown. This eliminates a separate cell-harvesting operation and also converts the cells into a more easily recoverable form. If harvesting of the cells before treatment to immobilise them is required, the cells can be collected and washed in the usual manner before being re-suspended in an aqueous medium (preferably having a wet cell concentration of 2% to 10%) and then treating with binding and flocculating agents.

The process of the invention for the isomerization of glucose to fructose is suitably carried out continuously by passing a glucose syrup through a suitable bed of the material. A batch process may also be used if desired. For a continuous process a suspension of the isomerase-containing material is first prepared by adding the material to a solution containing a magnesium salt and a suitable buffer which will maintain the solution at a pH of about 8 or 9. The magnesium salt increases the enzyme actively and the buffering agent removes any acidic materials which could cause enzyme denaturation. A solution which is 0.05 molar in respect of sodium bicarbonate and 0.01 molar in respect of magnesium chloride is satisfactory for preparing s suspension of the enzyme-containing material. When using dried material to prepare this suspension, the slurry is preferably allowed to stand for 30 to 60 minutes to allow the individual particles to swell. The suspension produced is poured carefully into a suitable column containing additional quantities of the sodium bicarbonate-magnesium chloride solution to avoid the entrapment of air bubbles. Continued treatment of the reactor bed with sodium bicarbonate-magnesium chloride solution for a period is desirable in order to attain maximum enzyme activity more quickly than is the case when a glucose solution is passed through the reactor bed immediately after the preparation thereof. The period of this pretreatment depends on the size of the reactor bed and on the flow rate of the solution through the bed. Generally a volume of solution equivalent to at least twice the volume of the reactor bed is passed through the bed to obtain the optimum effect.

The dimensions of the reactor bed used in the process of the invention for isomerizing glucose to fructose depend upon the substrate flow rate and upon the degree of isomerization required. The bed depth should be sufficient to minimise channelling. For columns of circular cross section it is preferred to have a depth to diameter ratio of at least one. Most conveniently the glucose syrup is allowed to flow through the bed by gravity. Upward or horizontal flow systems may also be used if required. The

apparatus containing the reactor bed should be equipped with suitable heating means to enable the glucose-isomerase containing material to be maintained at temperatures above ambient temperature, preferably between 50° and 75°C.

The concentration of glucose feed stock used in the process is not critical. Preferably a purified dextrose solution of 30 to 60% by weight or a high DE (i.e. DE greater than 94) starch conversion syrup with a solids content of 30% to 50% by weight is used as the feed.

The invention is illustrated by the following Examples:-

EXAMPLE 1

The polycationic flocculating agent "Primafloc C-7" and the polyanionic flocculating agent "Primafloc A10" which were used in this example were obtained from Rohm & Haas Corp. The bacterium used as the source of the glucose isomerase to be immobilised was *Arthrobacter* strain NRRL B-3728.

Cells of *Arthrobacter* strain NRRL B-3728 were flocculated by the addition with stirring of 0.25 g of "Primafloc C-7" per g dry weight of cells. To the thus flocculated cells was added 0.05 g of "Primafloc A10" per g dry weight of cells. The flocculated cells were then harvested by filtration through a cloth gauze and were partially dried for 10 minutes at a temperature of 60°C in a fluidised bed drier. The partially dried cells thus produced were extruded through a 1 mm aperture using an "Instron" 1195 extruder and were then further dried for 45 minutes at 60°C in the fluidised bed drier. The dried cells thus produced were ground using a coffee grinder, sieved and the size fraction in the range 1000 to 1180 μ was collected and was used as the immobilised enzyme.

This immobilised enzyme retained its structural integrity when stirred in a 40% glucose solution at 60°C for 16 hours.

EXAMPLE 2

The polycationic flocculating agent "Decapol X3000" used in this example was obtained from Fospur Ltd.

Immobilised glucose isomerase enzyme was prepared as

described in Example 1 except that 0.25 g of "Decapol X3000" per g dry weight of cells was used in place of "Primafloc C-7" as the polycationic flocculating agent. However the immobilised enzyme prepared in this way disintegrated when it was stirred in a 45% $^{w}/w$ glucose solution at a temperature of 60°C for 16 hours. Moreover the addition of extra "Decapol X3000" and/or of the polyanionic flocculating agent "Primafloc A10" to the cells during the flocculation stage of the process did not improve the structural characteristics of the immobilised enzyme. This result indicates that X3000 cannot be used effectively as a direct replacement for "Primafloc C-7" as the polycationic flocculating agent.

EXAMPLE 3

Immobilised glucose isomerase enzyme was prepared as described in Example 2 except that 0.25 g of casein per g dry weight of cells was added to the culture prior to flocculation. Moreover during the flocculation stage of the process the amounts of the flocculating agents added were increased to take account of the addition of casein. 0.25 g of "Decapol X3000" and 0.05 g of "Primafloc A10" were added per g dry weight of cells and per g of casein in the culture. The immobilised enzyme made in this way retained its structure when stirred in a 40% glucose solution at a temperature of 60°C for 16 hours. This result contrasts with that obtained in Example 2 and indicates that the presence of casein improves the structural properties of immobilised enzyme aggregates made with "Decapol X3000" and "Primafloc A10".

EXAMPLE 4

Immobilised glucose isomerase enzyme was prepared as described in Example 3 except that 0.5 g of casein per g dry weight of cells was added to the culture prior to flocculation. The immobilised enzume which was produced retained its structural integrity when stirred in a 40% glucose solution at a temperature of 60°C for 16 hours.

EXAMPLE 5

Immobilised glucose isomerase enzyme was prepared as

described in Example 4 except that glutaraldehyde was added to the culture after flocculation with "Decapol X3000" and "Primafloc A10" in an amount sufficient to give a glutaraldehyde concentration in the final solution of 0.2% by weight. The immobilised enzyme made in this way retained its structural integrity when stirred in a 40% glucose solution at a temperature of 60°C for 16 hours.

EXAMPLE 6

Immobilised glucose isomerase enzyme samples made as described in Examples 1, 3, 4 and 5 were assayed for glucose isomerase activity. The results are set out in the Table where activities are expressed as activities per g dry weight of cells in the immobilised enzyme product. An arbitrary figure of 100 was set for the activity of the conventional product made as described in Example 1 using "Primafloc C-7" and "Primafloc A10". It can be seen from the results that the enzyme products of Examples 3, 4 and 5, made with casein by the method of the invention, have significantly higher activities than the product of Example 1 made with "Primafloc C-7" and "Primafloc A10" alone. Thus the use of Casein as a binding agent in addition to improving the structural characteristics of immobilised enzyme products made with "Decapol X3000", an alternative to "Primafloc C-7" as the polycationic flocculating agent, also improves the enzyme activities exhibited by the immobilised enzyme products. We believe that the improved enzyme activities of the products are due to improvements in the diffusion characteristics of the products attributable to the use of casein.

Table

| Immobilised enzyme product | Enzyme Activity |
|---|---|
| Example 1 - "Primafloc C-7"/"Primafloc A10" | 100 |
| Example 3 - "Decapol X3000"/"Primafloc A10"/ casein | 124 |
| Example 4 - "Decapol X3000"/"Primafloc A10"/ casein | 136 |
| Example 5 - "Decapol X3000"/"Primafloc A10"/ casein/glutaraldehyde | 123 |

In the above Examples the structural strengths of the immobilised enzyme aggregates produced were demonstrated by a test in which the aggregates were stirred in 40% glucose solutions at a temperature of 60°C for 16 hours. It was found that the aggregate prepared conventionally using flocculating agents "Primafloc C-7" and "Primafloc A10" retained its structural integrity after this test which was then used to compare the comparative strengths of the immobilised enzyme aggregates of the invention with this prior art aggregate. The ability of an immobilised enzyme aggregate to retain its structural integrity for a lengthy period is important if it is to be used in a column reaction.

EXAMPLE 7

Immobilisation on plant scale without binding agent

Cells of *Arthrobacter* strain NRRL B-3728 were produced in a plant of commercial size (2 $M^3$ scale) and were passed into a tank to be immobilised as follows using conventional means. In the culture produced the concentration of cells was 16.2 g/l dry weight.

The polycationic flocculating agent used in this Example was 733/DPL obtained from Fospur Ltd. The polyanionic flocculating agent used was the same as in Example 1.

The cells were flocculated by the addition with stirring

of 0.25 g of 733/DPL per g dry weight of cells. The 733/DPL was added as a 2% solution in water. To the thus flocculated cells was added 0.25 g of "Primafloc A10" per g dry weight of cells. The "Primafloc A10" was added as a 5% aqueous solution. 50% glutaraldehyde solution was then added as an adhesive material to a final concentration of 0.2%. The flocculated cells were then harvested by centrifugation using a commercial decanter separator, and were partially dried for 10 minutes at a temperature of 60°C. The partially dried cells thus produced were extruded using an extruder fitted with a 1.2 mm foil and were then further dried for a period between 60 and 90 minutes at 60°C. The dried cells thus produced were ground, sieved and the size fraction in the range 683 to 1568 μm was collected and was used as the immobilised enzyme.

The enzyme activity of the product measured by standard procedures was as follows:-

Normal Product activity = 240 GI units/g of product

NB. A GI unit is defined as 1 μ mole of glucose isomerized per minute.

## EXAMPLE 8

Immobilisation on plant scale with binding agent

Example 7 was repeated using culture having a cell dry weight of 14 g/l and using casein as a binding agent during the flocculation treatment. Casein was added before the addition of the flocculating agents to a concentration of 0.4 g per g dry weight of cells.

The enzyme activity of the product measured by standard procedures was as follows:-

Normal product activity = 255 GI units/g of product.

The activity of the enzyme immobilised according to the invention in this Example is thus greater than that of the enzyme immobilised without use of a binding agent in Example 7. However the above figures do not give an exact comparison and under-state the extent of the improvement achieved by use of the method of the invention. The product of Example 8 contains 40% of casein which will not

contribute to the enzyme activity. If compensation is made for this an activity of the order of 425 GI units/g is obtained for the product of Example 8.

EXAMPLE 9

Cells of Arthrobacter strain NRRL B-3728 were produced in a semi-technical scale plant of 1 $M^3$ capacity. The culture produced contained 10 g/l dry weight of cells and was flocculated using the conditions of Example 8 with the minor differences that 733/DPL was added as a 0.5% aqueous solution to a concentration of 0.27 g/g dry weight of cells and "Primafloc A10" was added to a final concentration of 0.27 g/g dry weight of cells.

The enzyme activity of the product measured by standard procedures was as follows:-

Normal product activity = 237 GI units/g of product

NB. as with the product of Example 8 allowance should be made for the presence of casein.

70 g of the product was packed into a column which was used to continuously isomerize glucose to fructose using standard conditions. The column performed satisfactorily for the duration of the test which was 720 hours.

PA/JNA/MP
17 January 1984

1. A method for the production of an immobilised enzyme in which microbial cells containing the enzyme are formed into an aggregate by treating the cells with a water insoluble binding agent having a particle size within the range 10 to 500 µ diameter and thereafter with a polycationic flocculating agent and a polyanionic flocculating agent followed by recovery of aggregate particles.

2. A method according to claim 1 wherein the enzyme is glucose isomerase.

3. A method according to claim 1 or claim 2 wherein the binding agent is casein.

4. A method according to any one of the preceding claims wherein the binding agent is added to a culture containing the cells in an amount within the range 20% to 50% by weight of the dry weight of the cells in the culture.

5. A method according to any one of the preceding claims wherein an adhesive material which is a compound having a molecular weight not greater than 1000 and having a plurality of functional groups is added to a culture containing the cells after the addition thereto of the binding agent and the polycationic and polyanionic flocculating agents.

6. A method according to claim 5 wherein the adhesive material is glutaraldehyde.

7. An immobilised enzyme in aggregate particles comprising microbial cells containing the enzyme, said particles being formed by treating the cells with a water insoluble binding agent having a particle size within the range 10 to 500 µ diameter and thereafter with a polycationic flocculating agent and a polyanionic flocculating agent followed by recovery of aggregate particles.

8. An immobilised enzyme according to claim 7 wherein the enzyme immobilised in the aggregate is glucose isomerase, lactase, amyloglucosidase or cyanide hydrolase.

9. A process for performing an enzyme-catalysed reaction in the presence of aggregate particles comprising microbial cells containing the enzyme which catalyses the reaction, the aggregate

particles having been formed by treating the cells with a water insoluble binding agent having a particle size within the range 10 to 500 µ diameter and thereafter with a polycationic flocculating agent and a polyanionic flocculating agent followed by recovery of aggregate particles, the process comprising contacting the aggregate particles with a reactant under conditions suitable for performing the enzyme catalysed reaction and recovering a product of the reaction.

10. A process according to claim 9 wherein glucose is isomerised to fructose by contacting aggregate particles comprising glucose isomerase-containing microbial cells with a glucose solution at a temperature in the range $50^{\circ}$ to $90^{\circ}$ and at a pH in the range 6 to 10 to effect isomerization of at least a portion of the glucose in the solution.

European Patent Office

# EUROPEAN SEARCH REPORT

0118979
Application number

EP 84300444.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y,D | US - A - 4 060 456 (LONG)<br>* Claims 1,5,6; example 1 *<br>-- | 1,2,7-10 | C 12 N 11/02<br>C 12 N 11/00<br>C 12 P 19/24 |
| Y | US - E - 29 130 (LEE et al.)<br>* Claims 1,2,14,15 *<br>-- | 1,2,7-10 | |
| Y | US - A - 3 980 521 (AMOTZ et al.)<br>* Claim 1 *<br>-- | 5,6 | |
| A | GB - A - 1 571 987 (NOVO INDUSTRI A/S)<br>* Claims 1,9 *<br>---- | 1,3,5-8 | |

TECHNICAL FIELDS SEARCHED (Int. Cl. 3)

C 12 N
C 12 P

The present search report has been drawn up for all claims

| Place of search | Date of completion of the search | Examiner |
|---|---|---|
| VIENNA | 17-05-1984 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document